# FASCICULE DE BREVET EUROPEEN

(11) **EP 1 343 459 B1**
(45) Date de publication et mention de la délivrance du brevet: **03.01.2007**
(21) Numéro de dépôt: 01270193.4
(22) Date de dépôt: 20.11.2001
(51) Int. Cl.: A61Q 19/08, A61K 8/49, A61K 8/63, A61K 8/97

(54) **COMPOSITION, NOTAMMENT COSMETIQUE, RENFERMANT LA 7-HYDROXY DHEA ET/OU LA 7-CETO DHEA ET AU MOINS UN ISOFLAVONOIDE**
ZUSAMMENSETZUNG, INSBESONDERE KOSMETISCHE ZUSAMMENSETZUNG, MIT 7-HYDROXY-DHEA UND/ODER 7-KETO-DHEA UND ZUMINDEST EINEM ISOFLAVONOID
COMPOSITION, IN PARTICULAR COSMETIC, CONTAINING 7-HYDROXY DHEA AND/OR 7-KETO DHEA AND AT LEAST ONE ISOFLAVONOID

(30) Priorité: 15.12.2000 FR 0016432
(43) Date de publication de la demande: 17.09.2003
(73) Titulaire: L'ORÉAL, 75008 Paris (FR)
(72) Inventeur: BRETON, Lionel, F-78000 Versailles (FR)
(74) Mandataire: Renard, Emmanuelle
(86) Numéro de dépôt international: PCT/FR2001/003642
(87) Numéro de publication internationale: WO 2002/047631

(56) Documents cités:
- EP-A- 0 908 183
- WO-A-00/01351
- WO-A-00/28996
- WO-A-92/03925
- WO-A-97/03676
- WO-A-98/40074
- WO-A-98/56373
- WO-A-99/07381
- DE-A- 4 432 947
- FR-A- 2 765 803
- FR-A- 2 777 182

## Description

La présente invention se rapporte à une composition renfermant la 7-hydroxy DHEA et/ou la 7-céto DHEA et au moins un isoflavonoïde, ainsi qu'à l'utilisation de ladite composition, notamment pour prévenir ou traiter les signes du vieillissement cutané actinique.

La DHEA, ou déhydroépiandrostérone, est un stéroïde naturel produit essentiellement par les glandes corticosurrénales. La DHEA exogène, administrée par voie topique ou orale, est connue pour sa capacité à promouvoir la kératinisation de l'épiderme (JP-07 196 467) et à traiter les peaux sèches en augmentant la production endogène et la sécrétion de sébum et en renforçant ainsi l'effet barrière de la peau (US-4,496,556). Il a également été décrit dans le brevet US-5,843,932 l'utilisation de la DHEA pour remédier à l'atrophie du derme par inhibition de la perte de collagène et de tissu conjonctif. Enfin, la Demanderesse a mis en évidence la capacité de la DHEA à lutter contre l'aspect papyracé de la peau (FR 00/00349), à moduler la pigmentation de la peau et des cheveux (FR 99/12773) et à lutter contre l'atrophie de l'épiderme (FR 00/06154). Ces propriétés de la DHEA en font un candidat de choix comme actif anti-âge.

Parmi les métabolites de la DHEA, une attention particulière a été portée ces dernières années à la 7α-hydroxy DHEA. Il a en effet été démontré que ce métabolite, qui ne possède pas l'activité hormonale de la DHEA, permettait d'augmenter la prolifération des fibroblastes et la viabilité des kératinocytes humains et présentait des effets anti-radicalaires (WO 98/40074). Il a également été mis en évidence sur le rat (WO 00/28996) que la 7α-hydroxy DHEA augmentait l'épaisseur du derme et le contenu en élastine et collagène de la peau. Il a ainsi été suggéré d'utiliser ce métabolite de DHEA pour prévenir et/ou traiter les effets néfastes des UV sur la peau, lutter contre les rides et augmenter la fermeté et la tonicité de la peau.

La 7α-hydroxy DHEA est, avec le 5-androstène 3β,17β-diol, un métabolite majeur de la DHEA, obtenu par action de la 7α-hydroxylase sur la DHEA. Parmi les métabolites mineurs de la DHEA, on peut citer la 7β-hydroxy DHEA, obtenue par action de la 7β-hydroxylase sur la DHEA et la 7-céto DHEA, qui est elle-même un métabolite de la 7β-hydroxy DHEA.

Dans la suite de cette description, l'expression "7-hydroxy DHEA" sera utilisée pour désigner indifféremment la 7α-hydroxy DHEA et la 7β-hydroxy DHEA.

Il est maintenant apparu à la Demanderesse que l'association de la 7-hydroxy DHEA et/ou de la 7-céto DHEA avec un isoflavonoïde permettait de prévenir ou traiter plus efficacement les signes de vieillissement cutané, en particulier du vieillissement actinique ou photovieillissement.

La présente invention a donc pour objet une composition renfermant, dans un milieu physiologiquement acceptable : (a) au moins un dérivé de DHEA choisi parmi la 7-hydroxy DHEA et la 7-céto DHEA, et (b) au moins un isoflavonoïde.

La 7-hydroxy DHEA est de préférence la 7α-OH DHEA. Un procédé de préparation de ce composé est notamment décrit dans les demandes de brevet FR-2 771 105 et WO 94/08588. Toutefois, la 7β-OH DHEA convient également à une utilisation dans la présente invention.

La concentration en dérivé de DHEA dans la composition selon l'invention est avantageusement comprise entre 0,0000001 % et 10% en poids, de préférence entre 0,00001 % et 5% en poids, par rapport au poids total de la composition.

La composition selon la présente invention renferme, en association avec le dérivé de DHEA, au moins un isoflavonoïde.

Les isoflavonoïdes constituent une sous-classe des flavonoïdes, formés d'un squelette 3-phényl chromane qui peut comporter des substituants variés et différents niveaux d'oxydation. Contrairement aux flavonoïdes, ils ne sont présents que dans un nombre très limité de plantes.

Le terme isoflavonoïde regroupe plusieurs classe de composés parmi lesquels on peut citer les isoflavones, les isoflavanones, les roténoïdes, les pterocarpans, les isoflavanes, les isoflavanes-3-enes, les 3-arylcoumarines, les 3-aryl-4-hydroxycoumarins, les coumestanes, les coumaronochromones ou encore les 2-arylbenzofuranes. A cet égard on ce reportera avantageusement pour une revue complète sur les isoflavonoïdes, leurs méthodes d'analyse et leurs sources, au chapitre 5 "Isoflavonoids" écrit par P.M. Dewick, dans The Flavonoïds, Harbone éditeur, pp. 125-157 (1988).

Les isoflavonoïdes convenant à une mise en oeuvre dans la présente invention peuvent être d'origine naturelle ou synthétique. Par "origine naturelle", on entend l'isoflavonoïde à l'état pur ou en solution à différentes concentrations, obtenu par différents procédés d'extraction à partir d'un élément, généralement une plante, d'origine naturelle. Par "origine synthétique", on entend l'isoflavonoïde à l'état pur ou en solution à différentes concentrations, obtenu par synthèse chimique.

On préfère utiliser les isoflavonoïdes d'origine naturelle. Parmi ceux-ci, on peut citer : la daidzine, la génistine, la daidzéine, la formononétine, la cunéatine, la génistéine, l'isoprunétine et la prunétine, la cajanine, l'orobol, la pratenséine, le santal, la junipégénine A, la glycitéine, l'afrormosine, la rétusine, la tectorigénine, l'irisolidone, la jamaicine, ainsi que leurs analogues et métabolites.

L'isoflavonoïde représente de préférence de 10⁻¹⁰% à 10%, et de préférence de 10⁻⁸ à 5%, du poids total de la composition. Bien entendu, si l'isoflavonoïde est présent sous forme de solution contenant un extrait de plante, l'homme du métier saura ajuster la quantité de cette solution dans la composition selon l'invention, de façon à obtenir les gammes de concentrations ci-dessus en isoflavonoïde.

Les isoflavones sont préférées pour une utilisation dans la présente invention. Par ce terme, on entend aussi bien les formes aglycones (daidzéine, génistéine, glycitéine) que les formes glycosylées (daidzine, génistine, glycitine) des isoflavones.

Des procédés de préparation d'isoflavones sont notamment décrits dans WO 95/10530, WO 95/10512, US-5,679,806, US-5,554,519, EP-812 837 et WO 97/26269.

Toutefois, pour une utilisation dans la présente invention, on préfère utiliser les isoflavones sous forme d'extraits de graines de soja commercialisés par ICHIMARU PHARCOS sous la dénomination commerciale Flavostérone SB^{®} et par ARCHER DANIELS MIDLAND COMPANY sous la dénomination commerciale Novasoy^{®}. Ces extraits renferment majoritairement des isoflavones sous forme glycosylée, susceptibles d'être métabolisées dans l'organisme en leurs formes aglycones, et minoritairement des isoflavones aglycones.

Les isoflavones sont en particulier connues en tant qu'anti-oxydants, pour leurs propriétés anti-radicalaires et dépigmentantes, ainsi que pour inhiber l'activité des glandes sébacées (voir notamment DE-44 32 947). Elles ont également été décrites en tant qu'agents pour prévenir les signes du vieillissement de la peau, dont le relâchement cutané et la perte d'éclat du teint (JP 1-96106).

On comprend donc que l'association d'un dérivé de DHEA tel que défini précédemment avec des isoflavonoïdes permet de renforcer les effets anti-âge de la composition les contenant, en particulier lorsqu'il s'agit de prévenir ou traiter les signes du vieillissement cutané actinique.

La composition selon l'invention est de préférence adaptée à une application topique sur la peau. Elle peut se présenter sous toutes les formes galéniques normalement utilisées pour ce type d'application, notamment sous forme d'une solution aqueuse ou huileuse, d'une émulsion huile-dans-eau ou eau-dans-huile ou multiple, d'une émulsion siliconée, d'une microémulsion ou nanoémulsion, d'un gel aqueux ou huileux ou d'un produit anhydre liquide, pâteux ou solide.

Cette composition peut être plus ou moins fluide et avoir l'aspect d'une crème blanche ou colorée, d'une pommade, d'un lait, d'une lotion, d'un sérum, d'une pâte, d'une mousse ou d'un gel. Elle peut éventuellement être appliquée sur la peau sous forme d'aérosol. Elle peut également se présenter sous forme solide, et par exemple sous forme de stick. Elle peut être utilisée comme produit de soin et/ou comme produit de maquillage de la peau.

De façon connue, la composition de l'invention peut contenir également les adjuvants habituels dans les domaines cosmétique et dermatologique, tels que les gélifiants hydrophiles ou lipophiles, les actifs hydrophiles ou lipophiles, les conservateurs, les antioxydants, les solvants, les parfums, les charges, les filtres, les pigments, les absorbeurs d'odeur et les matières colorantes. Les quantités de ces différents adjuvants sont celles classiquement utilisées dans les domaines considérés, et par exemple de 0,01 à 20 % du poids total de la composition. Ces adjuvants, selon leur nature, peuvent être introduits dans la phase grasse ou dans la phase aqueuse. Ces adjuvants, ainsi que leurs concentrations, doivent être tels qu'ils ne nuisent pas aux propriétés avantageuses des dérivés de DHEA, ni des isoflavonoïdes selon l'invention.

Lorsque la composition selon l'invention est une émulsion, la proportion de la phase grasse peut aller de 5 à 80 % en poids, et de préférence de 5 à 50 % en poids par rapport au poids total de la composition. Les matières grasses, les émulsionnants et les coémulsionnants utilisés dans la composition sous forme d'émulsion sont choisis parmi ceux classiquement utilisés dans le domaine considéré. L'émulsionnant et le coémulsionnant sont de préférence présents, dans la composition, en une proportion allant de 0,3 à 30 % en poids, et de préférence de 0,5 à 20 % en poids par rapport au poids total de la composition.

Comme matières grasses utilisables dans l'invention, on peut utiliser les huiles et notamment les huiles minérales (huile de vaseline), les huiles d'origine végétale (huile d'avocat, huile de soja), les huiles d'origine animale (lanoline), les huiles de synthèse (perhydrosqualène), les huiles siliconées (cyclométhicone) et les huiles fluorées (perfluoropolyéthers). On peut aussi utiliser comme matières grasses des alcools gras tels que l'alcool cétylique, des acides gras, des cires et des gommes et en particulier les gommes de silicone.

Comme émulsionnants et coémulsionnants utilisables dans l'invention, on peut citer par exemple les esters d'acide gras et de polyéthylène glycol tels que le stéarate de PEG-100, le stéarate de PEG-50 et le stéarate de PEG-40 ; les esters d'acide gras et de polyol tels que le stéarate de glycéryle, le tristéarate de sorbitane et les stéarates de sorbitane oxyéthylénés disponibles sous les dénominations commerciales Tween^{®} 20 ou Tween^{®} 60, par exemple ; et leurs mélanges.

Comme gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles, et, comme gélifiants lipophiles, on peut citer les argiles modifiées comme les bentones, les sels métalliques d'acides gras et la silice hydrophobe.

Selon une variante de l'invention, la composition peut être adaptée à une administration par voie orale. Dans ce cas, elle peut se présenter sous forme de sirops, de suspensions, de solutions, d'émulsions, de granules, de capsules ou de comprimés, par exemple,

Les doses quotidiennes de dérivé de DHEA administrées par voie orale peuvent être comprises entre 1 et 100 mg/jour, de préférence entre 25 et 75 mg/jour. Préférentiellement, le dérivé de DHEA est présent dans la composition selon l'invention en une quantité permettant son administration à une dose comprise entre 50 et 100 mg/jour, ladite posologie étant réalisée en une ou plusieurs prises, avec une dose unitaire de 50 mg.

Les doses quotidiennes d'isoflavonoïdes administrées par voie orale sont à définir au cas par cas pour obtenir l'effet physiologique recherché. Plus particulièrement s'il s'agit d'isoflavones, seules ou dans un extrait, les doses journalières en isoflavones peuvent être comprises entre 0,1 et 500 mg/jour. Préférentiellement, les isoflavones sont présents dans la composition selon l'invention en une quantité permettant son administration à une dose comprise entre 10 et 300 mg/jour. Ladite posologie étant réalisée en une ou plusieurs prises.

Dans tous les cas, la composition selon l'invention et/ou la préparation obtenue à partir de celle-ci comprend une quantité efficace de dérivé de DHEA et une quantité efficace d'isoflavonoïde, suffisante pour obtenir l'effet recherché, dans un milieu physiologiquement acceptable.

La composition selon l'invention trouve notamment une application dans la prévention et le traitement des signes du vieillissement cutané .

La présente invention concerne donc également l'utilisation cosmétique de la composition mentionnée ci-dessus pour prévenir ou traiter les signes du vieillissement cutané

Elle concerne en particulier l'utilisation cosmétique de la composition décrite précédemment pour prévenir ou lutter contre la formation de rides et/ou pour améliorer la tonicité de la peau et/ou pour augmenter la fermeté de la peau.

Elle se rapporte enfin à l'utilisation de la composition décrite précédemment pour fabriquer une préparation destinée à prévenir ou lutter contre les effets néfastes des UV sur la peau.

L'invention sera maintenant illustrée par les exemples non limitatifs suivants. Dans ces exemples, les quantités sont indiquées en pourcentage pondéral.

### Exemple 1 : composition pour application topique

| *Phase A1* | | |
|---|---|---|
| 2-octyldodécanol | | 20 % |
| 7α-OH DHEA | | 1 % |
| | | |

| *Phase A2* | | |
|---|---|---|
| Distéarate polyglycérolé (2 mol) | | 2 % |
| Mono-stéarate de PEG (8 OE) | | 1,35% |
| Acide stéarique | | 1 % |
| Conservateur | | 0,1 % |
| | | |

| *Phase B* | | |
|---|---|---|
| Conservateurs | | 0,35 % |
| Neutralisants | | 0,25% |
| Propylène glycol | | 5 % |
| Extrait aqueux de graines de soja contenant 0,2 % d'isoflavones et 30 % de butylène glycol (Flavostérone SB - ICHIMARU PHARCOS) | | 5 % |
| Eau | qsp | 100 % |

| *Phase C* | | |
|---|---|---|
| Gélifiant | | 0,5 % |
| Neutralisant | | 0,2 % |
| Eau | | qsp |

Cette composition peut être préparée de la manière suivante : les phases A1, A2 et B sont préparées séparément par mélange de leurs constituants à chaud, sous agitation. Les phases A1 et A2 sont mélangées à chaud, puis la phase B leur est ajoutée. Après refroidissement à température ambiante, la phase C est ajoutée au mélange ainsi obtenu.

Cette composition peut être utilisée en applications bi-quotidiennes pour prévenir ou traiter les signes du vieillissement cutané tels que les rides et le relâchement cutané.

### Exemple 2 - Composition pour administration orale

On prépare, de manière classique pour l'homme du métier, des capsules molles ayant la composition suivante :

| | |
|---|---|
| Huile de soja hydrogénée | 40 mg |
| Huile de blé | 95 mg |
| Lécithine de soja | 20 mg |
| Tocophérols naturels | 5 mg |
| Extrait de soja disponible sous la dénomination Novasoy^{®} | |
| auprès de la société AMD (correspondant à environ 50 mg d'isoflavones) | 120 mg |
| 7α-OH DHEA | 50 mg |

## Revendications

1. Composition renfermant, dans un milieu physiologiquement acceptable : (a) au moins un dérivé de DHEA choisi parmi la 7-hydroxy DHEA et la 7-céto DHEA, et (b) au moins un isoflavonoïde.

2. Composition selon la revendication 1, **caractérisée en ce que** la 7-hydroxy DHEA est la 7α-OH DHEA.

3. Composition selon la revendication 1, **caractérisée en ce que** la 7-hydroxy DHEA est la 7β-OH DHEA.

4. Composition selon l'une quelconque des revendications 1 à 3, **caractérisée en ce que** ledit isoflavonoïde est choisi parmi les isoflavonoïdes d'origine naturelle.

5. Composition selon la revendication 4, **caractérisée en ce que** ledit isoflavonoïde est choisi parmi les isoflavones.

6. Composition selon la revendication 4 ou 5, **caractérisée en ce que** ledit isoflavonoïde est choisi parmi : la daidzine, la génistine, la daidzéine, la formononétine, la cunéatine, la génistéine, l'isoprunétine et la prunétine, la cajanine, l'orobol, la pratenséine, le santal, la junipégénine A, la glycitéine, l'afrormosine, la rétusine, la tectorigénine, l'irisolidone, la jamaicine, ainsi que leurs analogues et métabolites.

7. Composition selon la revendication 6, **caractérisée en ce que** ledit isoflavonoïde est choisi parmi la daidzéine, la génistéine, la glycitéine, la daidzine, la génistine et la glycitine.

8. Composition selon l'une quelconque des revendications 1 à 7, **caractérisée en ce qu'**elle est adaptée à une application topique sur la peau.

9. Composition selon la revendication 8, **caractérisée en ce qu'**elle renferme de 0,0000001 à 10% en poids de dérivé de DHEA, par rapport au poids total de la composition.

10. Composition selon la revendication 9, **caractérisée en ce qu'**elle renferme de 0,00001, à 5% en poids de dérivé de DHEA, par rapport au poids total de la composition.

11. Composition selon l'une quelconque des revendications 8 à 10, **caractérisée en ce qu'**elle comprend de 10⁻¹⁰% à 10% en poids d'isoflavonoïde, par rapport au poids total de la composition.

12. Composition selon la revendication 11, **caractérisée en ce qu'**elle comprend de 10⁻⁸% à 5% en poids d'isoflavonoïde, par rapport au poids total de la composition.

13. Composition selon l'une quelconque des revendications 1 à 7, **caractérisée en ce qu'**elle est adaptée à une administration par voie orale.

14. Utilisation cosmétique de la composition selon l'une quelconque des revendications 1 à 13 pour prévenir ou traiter les signes du vieillissement cutané

15. Utilisation cosmétique de la composition selon l'une quelconque des revendications 1 à 13 pour prévenir ou lutter contre la formation de rides et/ou pour améliorer la tonicité de la peau et/ou pour augmenter la fermeté de la peau.

16. Utilisation de la composition selon l'une quelconque des revendications 1 à 13 pour fabriquer une préparation destinée à prévenir ou lutter contre les effets néfastes des UV sur la peau.

## Claims

1. Composition containing, in a physiologically acceptable medium: (a) at least one DHEA derivative chosen from 7-hydroxy-DHEA and 7-keto-DHEA, and (b) at least one isoflavonoid.

2. Composition according to Claim 1, **characterized in that** the 7-hydroxy-DHEA is 7α-OH-DHEA.

3. Composition according to Claim 1, **characterized in that** the 7-hydroxy-DHEA is 7β-OH-DHEA.

4. Composition according to any one of Claims 1 to 3, **characterized in that** said isoflavonoid is chosen from isoflavonoids of natural origin.

5. Composition according to Claim 4, **characterized in that** said isoflavonoid is chosen from isoflavones.

6. Composition according to Claim 4 or 5, **characterized in that** said isoflavonoid is chosen from daidzin, genistin, daidzein, formononetin, cuneatin, genistein, isoprunetin and prunetin, cajanin, orobol, pratensein, santal, junipegenin A, glycitein, afrormosin, retusin, tectorigenin, irisolidone, jamaicin, and their analogues and metabolites.

7. Composition according to Claim 6, **characterized in that** said isoflavonoid is chosen from daidzein, genistein, glycitein, daidzin, genistin and glycitin.

8. Composition according to any one of Claims 1 to 7, **characterized in that** it is suitable for topical application to the skin.

9. Composition according to Claim 8, **characterized in that** it contains from 0.0000001 to 10% by weight of DHEA derivative, relative to the total weight of the composition.

10. Composition according to Claim 9, **characterized in that** it contains from 0.00001 to 5% by weight of DHEA derivative, relative to the total weight of the composition.

11. Composition according to any one of Claims 8 to 10, **characterized in that** it comprises from 10⁻¹⁰% to 10% by weight of isoflavonoid, relative to the total weight of the composition.

12. Composition according to Claim 11, **characterized in that** it comprises from 10⁻⁸% to 5% by weight of isoflavonoid, relative to the total weight of the composition.

13. Composition according to any one of Claims 1 to 7, **characterized in that** it is suitable for administration by the oral route.

14. Cosmetic use of the composition according to any one of Claims 1 to 13 for the prevention or treatment of the signs of skin ageing.

15. Cosmetic use of the composition according to any one of Claims 1 to 13 for preventing or controlling the formation of wrinkles and/or for improving skin tone and/or for increasing skin firmness.

16. Use of the composition according to any one of Claims 1 to 13 for the manufacture of a composition intended for preventing or controlling the harmful effects of UV radiation on the skin.

## Patentansprüche

1. Zusammensetzung, die in einem physiologisch akzeptablen Medium (a) mindestens ein DHEA-Derivat, das unter 7-Hydroxy-DHEA und 7-Keto-DHEA ausgewählt ist, und (b) mindestens ein Isoflavonoid enthält.

2. Zusammensetzung nach Anspruch 1, **dadurch gekennzeichnet, dass** es sich bei dem 7-Hydroxy-DHEA um 7α-OH-DHEA handelt.

3. Zusammensetzung nach Anspruch 1, **dadurch gekennzeichnet, dass** das 7-Hydroxy-DHEA das 7β-OH-DHEA ist.

4. Zusammensetzung nach einem der Ansprüche 1 bis 3, **dadurch gekennzeichnet, dass** das Isoflavonoid unter den Isoflavonoiden natürlicher Herkunft ausgewählt ist.

5. Zusammensetzung nach Anspruch 4, **dadurch gekennzeichnet, dass** das Isoflavonoid unter den Isoflavonen ausgewählt ist.

6. Zusammensetzung nach Anspruch 4 oder 5, **dadurch gekennzeichnet, dass** das Isoflavonoid ausgewählt ist unter: Daidzin, Genistin, Daidzein, Formononetin, Cuneatin, Genistein, Isoprunetin und Prunetin, Cajanin, Orobol, Pratensein, Santal, Junipegenin A, Glycitein, Afrormosin, Retusin, Tectorigenin, Irisolidon, Jamaicin, sowie deren Analoga und Metaboliten.

7. Zusammensetzung nach Anspruch 6, **dadurch gekennzeichnet, dass** das Isoflavonoid unter Daidzein, Genistein, Glycitein, Daidzin, Genistin und Glycitin ausgewählt ist.

8. Zusammensetzung nach einem der Ansprüche 1 bis 7, **dadurch gekennzeichnet, dass** sie für eine topische Anwendung auf die Haut geeignet ist.

9. Zusammensetzung nach Anspruch 8, **dadurch gekennzeichnet, dass** sie 0,0000001 bis 10 Gew.-% DHEA-Derivat, bezogen auf das Gesamtgewicht der Zusammensetzung, enthält.

10. Zusammensetzung nach Anspruch 9, **dadurch gekennzeichnet, dass** sie 0,00001 bis 5 Gew.-% DHEA-Derivat, bezogen auf das Gesamtgewicht der Zusammensetzung, enthält.

11. Zusammensetzung nach einem der Ansprüche 8 bis 10, **dadurch gekennzeichnet, dass** sie 10-¹⁰ bis 10 Gew.-% Isoflavonoid, bezogen auf das Gesamtgewicht der Zusammensetzung, enthält.

12. Zusammensetzung nach Anspruch 11, **dadurch gekennzeichnet, dass** sie 10-⁸ bis 5 Gew.-% Isoflavonoid, bezogen auf das Gesamtgewicht der Zusammensetzung, enthält.

13. Zusammensetzung nach einem der Ansprüche 1 bis 7, **dadurch gekennzeichnet, dass** sie für eine Verabreichung auf oralem Weg geeignet ist.

14. Kosmetische Verwendung der Zusammensetzung nach einem der Ansprüche 1 bis 13 zur Vorbeugung oder Behandlung der Anzeichen der Hautalterung.

15. Kosmetische Verwendung der Zusammensetzung nach einem der Ansprüche 1 bis 13, um der Bildung von Falten vorzubeugen oder die Bildung von Falten zu bekämpfen und/oder den Tonus der Haut zu verbessern und/oder die Festigkeit der Haut zu erhöhen.

16. Verwendung der Zusammensetzung nach einem der Ansprüche 1 bis 13 für die Herstellung eines Präparats, das dazu vorgesehen ist, den schädlichen Wirkungen der UV-Strahlung auf der Haut vorzubeugen oder sie zu bekämpfen.
